# EUROPÄISCHE PATENTSCHRIFT

(11) **EP 1 791 584 B1**
(45) Veröffentlichungstag und Bekanntmachung des Hinweises auf die Patenterteilung: **20.01.2016**
(21) Anmeldenummer: 05786868.9
(22) Anmeldetag: 19.08.2005
(51) Int. Cl.: A61M 15/00

(54) **INHALATOR FÜR PULVERFÖRMIGE, INSBESONDERE MEDIZINISCHE SUBSTANZEN**
INHALER FOR POWDERED, PARTICULARLY MEDICAL SUBSTANCES
INHALATEUR POUR SUBSTANCES PULVÉRULENTES, EN PARTICULIER POUR SUBSTANCES MEDICALES

(30) Priorität: 27.08.2004 DE 102004041524
(43) Veröffentlichungstag der Anmeldung: 06.06.2007
(60) Teilanmeldung: 07118831.2
(73) Patentinhaber: Sanofi SA, 1214 Vernier (CH)
(72) Erfinder: Alfred von Schuckmann, 47627 Kevelaer (DE)
(74) Vertreter: Epping - Hermann - Fischer
(86) Internationale Anmeldenummer: PCT/EP2005/054094
(87) Internationale Veröffentlichungsnummer: WO 2006/021546

(56) Entgegenhaltungen:
- WO-A-00/64518
- DE-A1- 10 047 722
- DE-A1- 10 106 788
- DE-A1- 10 144 572
- US-A- 5 429 122

## Beschreibung

Die Erfindung betrifft einen Inhalator für pulverförmige, insbesondere medizinische Substanzen, mit einem zu einem Mundstück führenden Saugluftkanal, ferner einer Vorratskammer für die Substanz und einer linear sowie überlagert rotatorisch bewegten Dosierkammer zum Abteilen einer bestimmten Substanzmenge aus der Vorratskammer und Verbringen der Substanzmenge in eine Übergabeposition, zur Übergabe an den Saugluftstrom.

Ein Inhalator der in Rede stehenden Art ist aus der DE 10 144 572 A1 bekannt.

Aufgabe der Erfindung ist es, einen gattungsgemäßen Inhalator, insbesondere im Hinblick auf die Beladung der Dosierkammer durch Abteilen einer bestimmten Substanzmenge aus der Vorratskammer in vorteilhafter Weise weiterzubilden.

Diese Aufgabe ist durch den Gegenstand des Anspruchs 1 gelöst, wobei darauf abgestellt ist, dass die Dosierkammer in einem als Flachteil ausgeformten Tauchschieber ausgebildet ist und exzentrisch zu der Rotationsachse ihrer überlagerten rotatorischen Bewegung angeordnet ist. Zufolge solcher Ausgestaltung ist ein baulich einfacher, funktionssicherer Inhalator erzielt, dessen Dosierkammer durch die der Linearbewegung überlagerten rotatorischen Bewegung in einfacher und sicherer Weise befüllt wird, was noch dadurch weiter unterstützt ist, dass die Dosierkammer im Zuge der Rotationsbewegung mit radialem Abstand um die Rotationsachse durch die in der Vorratskammer bevorratete Substanz zur Abschöpfung einer vorbestimmten Substanzmenge bewegt wird. Es erfolgt entsprechend ein Abschöpfen einer stets gleich großen, vordefinierten Substanzmenge aus der Vorratskammer. Diese steht nach schraubengangförmiger Verlagerung der Dosierkammer in die Übergabeposition zur Übergabe an den Saugluftstrom und damit einhergehender Inhalation bereit.

Die Gegenstände der weiteren Ansprüche sind nachstehend im Bezug zu dem Gegenstand des Anspruchs 1 erläutert. Es wird weiter vorgeschlagen, dass die Dosierkammer bevorzugt als Querbohrung in dem als Flachteil ausgeformten Tauchschieber gestaltet ist. Als besonders vorteilhaft erweist sich hierbei, wenn, wie weiter bevorzugt, der Tauchschieber Verschlusskappen-abhängig verlagerbar ist, so dass in Ausübung der gewohnten Handhabung zum Schließen und Öffnen durch Verschlusskappendrehung zugleich eine Beladung der Dosierkammer und eine lineare, sowie überlagerte rotatorische Bewegung der Dosierkammer in die Übergabeposition erreicht wird. Eine besonders wirksame Maßnahme ergibt sich durch eine konische Querbohrung zur Ausformung der Dosierkammer. Das den Tauchschieber ausformende Flachteil weist im Querschnitt ein Kantenverhältnis von etwa 1:2 bis 1:5 auf. Das in Eintauchrichtung freie Ende dieses Flachteiles kann beispielsweise schraubendreherklingenartig ausgespitzt sein. Durch die flachteilartige Ausformung des Tauchschiebers ist ein Dreh-Auflockerungseffekt im Zentrumsbereich der in der in der Vorratskammer bevorrateten Substanz erreicht, was zugleich das Eintauchen des Tauchschiebers in den Pulversee begünstigt. Um einem Ausfallen der abgeschöpften Substanzmenge aus der Dosierkammer in der Übergabeposition vor einer Übergabe an den Saugluftstrom zu begegnen, ist in einer Weiterbildung des Erfindungsgegenstandes vorgesehen, dass der Tauchschieber außerhalb der Vorratskammer mit einem relativ zu dem Tauchschieber beweglichen Verschlusskolben zusammenwirkt. Bevorzugt ist dieser Verschlusskolben zu dem Tauchschieber relativ linear beweglich und entsprechend von dem als Flachteil ausgeformten Tauchschieber durchsetzt, was weiter die zueinander drehfeste Anordnung von Verschlusskolben und Tauchschieber bedingt. Zur Übergabe an der Substanzmenge an den Saugluftstrom ist der Verschlusskolben entsprechend zunächst in eine die Dosierkammer zur Substanzausgabe freigebende Stellung zu verlagern. Dies erfolgt bevorzugt dadurch, dass der Verschlusskolben abhängig von einem Saugunterdruck in eine Freigabestellung bezüglich der Dosierkammer zu bewegen ist. Der nötige Saugunterdruck wird im Zuge der Inhalation aufgebracht. Hierzu umfasst der Patient in für ihn bekannter Weise mit den Lippen das Mundstück des Inhalators um durch Einatmen einen Saugluftstrom in dem Saugluftkanal zu erzielen. Der hierbei automatisch aufgebaute Saugunterdruck reicht zur Verlagerung des Verschlusskolbens in die Freigabestellung und zum anschließenden Ausräumen der Dosierkammer und damit einhergehender Übergabe der Substanzmenge an den Saugluftstrom aus. Durch die Anordnung des Verschlusskolbens ist die Bevorratung der abgeschöpften Teilmenge in der Dosierkammer bis zur Inhalation, d.h. bis zur Übergabe an den Saugluftstrom gesichert. Die Freigabe bzw. das Öffnen der Dosierkammer erfolgt automatisch im Zuge der üblichen Handhabung. Um die Verteilung der pulverförmigen Substanz bzw. der abgeteilten Substanzmenge in der Saugluft weiter zu verbessern, ist der dem Übergabebereich in Strömungsrichtung nachgeschaltete, sogenannte Dispergiebereich dahingehend in vorteilhafter Weise weiter gebildet, dass der Saugluftkanal oberhalb der Dosierkammer eine Umlenkung nach radial außen aufweist. Demzufolge erfolgt vor Austritt der an den Saugluftstrom übergebenen Substanzmenge noch eine Umlenkung, wobei weiter diese nach radial außen weisende Umlenkung in einen ringraumartigen Austrittabschnitt im Bereich des Mundstückes führt. Entsprechend erfolgt der Substanzaustritt mit Bezug auf eine Draufsicht auf die Mundstückdecke kreisringförmig. Auch wird vorgeschlagen, dass der Tauchschieber selbst einen Teil der Strömungsumlenkung ausbildet, so insbesondere, in einem dem die Dosierkammer aufweisenden Flachteil abgewandten und entsprechend dem Mundstück zugewandten Endbereich in Form eines im Grundriss kreisrunden Tellers, welcher in der Übergabeposition die Umlenkung des Saugluftkanals nach radial außen bietet. Nachstehend ist die Erfindung der beigefügten Zeichnungen, welche lediglich ein Ausführungsbeispiel darstellt, näher erläutert. Es zeigt:

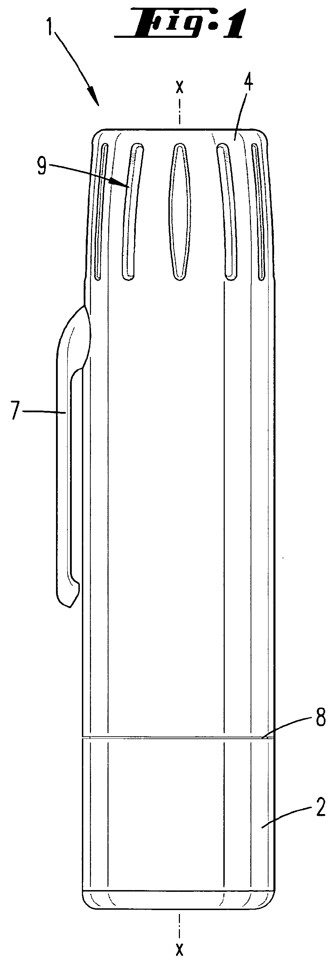

- Fig. 1: den erfindungsgemäßen Inhalator in Seitenansicht, vergrößert, in kappenverschlossener Grundstellung;
- Fig. 2: den Vertikalschnitt hierzu;

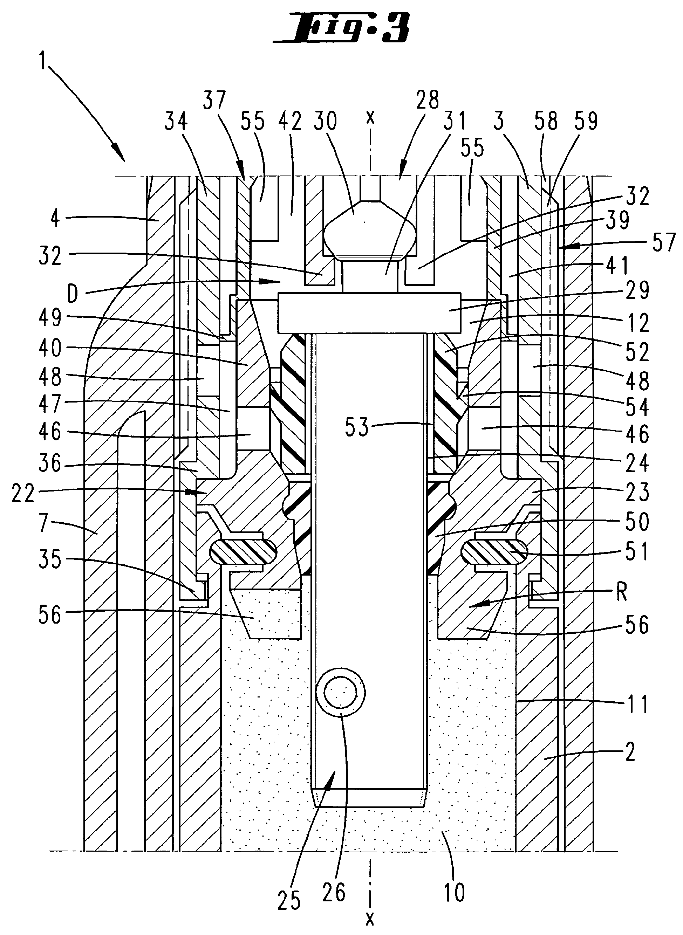

- Fig. 3: eine Herausvergrößerung aus Fig. 2, den Bereich einer Dosiervorrichtung betreffend;
- Fig. 4: eine Schnittdarstellung gemäß Fig. 2, jedoch bei abgenommener Verschlusskappe und hierdurch bedingter Verlagerung der Dosierkammer in die Endnahme-Bereitschaftstellung;
- Fig. 5: eine der Fig. 4 entsprechende Schnittdarstellung, jedoch eine Stellung im Zuge der Inhalation darstellend;

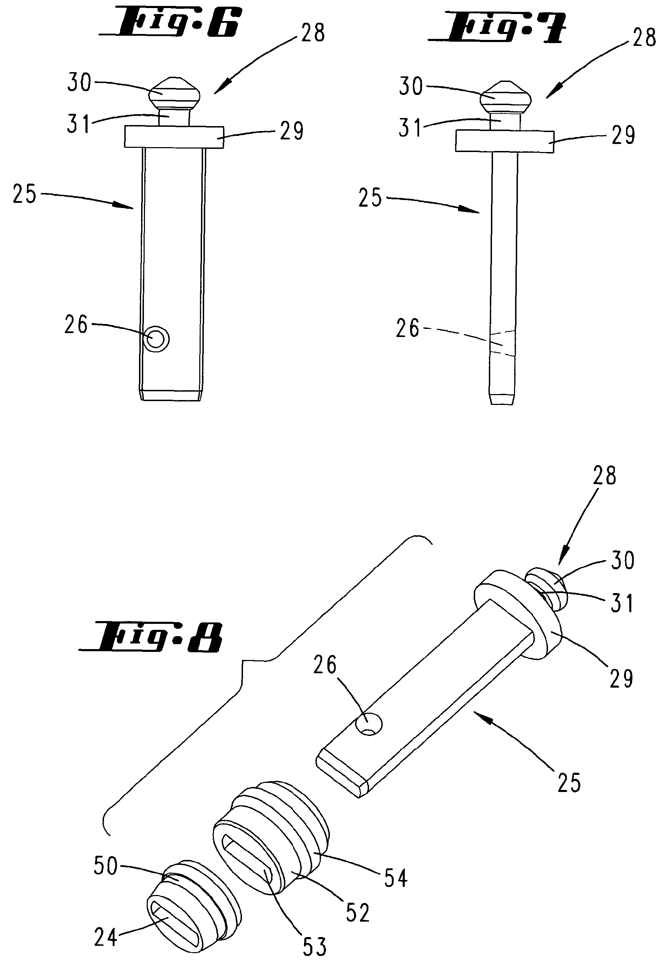

- Fig. 6: den die Dosierkammer aufweisenden Tauchschieber in einer Einzeldarstellung in Ansicht;
- Fig. 7: die Seitenansicht hierzu;
- Fig. 8: in einer perspektivischen Darstellung den Tauchschieber mit einem zuordbaren Verschlusskolben sowie einer Dichtbuchse.

Dargestellt und beschrieben ist ein Inhalator 1, welcher als bequem mitführbares, kurzstabförmiges Taschengerät realisiert ist, mit einem formbestimmenden abgesetzt, zylindrischen Gehäuse 2.

Das zylindrische, röhrchenartige Gehäuse 2 geht kopfseitig des Inhalators 1 in ein aufgesetztes Mundstück 3 über, welches mundgerecht abgeflacht ist und sich mittels einer becherförmigen Verschlusskappe 4 schützend überfangen lässt. Letztere ist als Schraubkappe realisiert, wozu ein ihr zugeordnetes Innengewinde 5 in ein korrespondierendes Außengewinde 6 an der Mantelwand des Gehäuses 2 eingreift. Im Ansatzbereich des Mundstückes 3 ist der Verschlusskappe 4 mantelaußenwandig ein Clip 7 angeformt.

Fußseitig tritt der Stirnrand der becherförmigen Verschlusskappe 4 anschlagbegrenzend und abdichtend gegen eine Ringschulter 8, welche aufgrund des vorgenannten Absatzes des zylindrischen Gehäuses 2 erzielt ist.

Die Verschlusskappe 4 dient zugleich als Betätigungshandhabe 9 zur Ausbringung einer pulverförmigen Substanz 10 in reproduzierbaren Teilmengen 10', wozu der axiale Schraubhub des Gewindeeingriffs 5/6 genutzt wird. Die Substanz 10 ist in einer Vorratskammer 11 des Gehäuses 2 gegebenenfalls nachfüllbar aufgenommen. Die jeweils eine Teilmenge 10' an eine außerhalb der Vorratskammer 11 liegende Übergangsstelle U fördernde Dosiervorrichtung ist in ihrer Ganzheit mit D bezeichnet.

Bezüglich des dosierfähigen Gutes handelt es sich um eine medizinische pulverförmige Substanz 10. So können beispielsweise saugstromtransportfähige Grundkörper wie Laktose Träger für oberflächenseitig anhaftende, mikronisierte Arzneimittel-Feinpartikel sein.

Der Dosiervorrichtung D ist nachgeschaltet ein sogenannter Dispergierbereich, in welchem durch den Benutzer ein Saugluftstrom S erzeugt wird. Dieser trägt die exakt abgeteilte Teilmenge 10' der Substanz 10 in der Übergabestelle U restfrei ab. Der zum Mundstück 3 leitende Saugluftkanal ist mit dem Bezugszeichen 12 versehen.

Den unteren Abschluss der Vorratskammer 11 bildet ein topfförmiger Druckboden 13, welcher in Richtung des Mundstückes 3 mittels einer Druckfeder 14 unter Federbelastung steht. Die Druckfeder 14 stützt sich mit der fußseitigen Endwindung an einer das Gehäuse 2 dort schließenden Bodenkappe 15 ab. Diese steht in Rasteingriff zum hier querschnittsgrößeren Abschnitt des Gehäuses 2, wobei ein entsprechender Rastkragen 16 der Bodenkappe 15 in eine passende Ringnut des Gehäuses 2 eingreift.

Die kopfseitige Endwindung der vorgespannten Druckfeder 14 wirkt belastend auf eine Innenschulter 17 eines Hohlkolbens 18 der kolbenförmigen Einrichtung 13/18. Wie aus den Darstellungen zu erkennen, ist der gestuft topfförmige Druckboden 13 mit der Innenschulter 17 des Hohlkolbens 18 rastverbunden.

Der Topfrand des Druckbodens 13 stellt eine Ringlippe 19, die aufgrund ihres gummielastischen Materials die Wandung der Vorratskammer 21 verlustfrei abstreift.

Von der Bodenkappe 15 geht zentral ein Hohl-Stehzapfen 20 aus. Dieser bildet zusammen mit dem diesen mit Abstand umgebenden Hohlkolben 18 eine Federkammer 21 für die Druckfeder 14.

Mundstückseitig schließt die Vorratskammer 11 mit einem topfförmigen Drehteil 22 ab, welches mit seinem Topfboden die das Gehäuse 2 überfangende Decke 23 der Vorratskammer 11 bildet.

Im Zentrum der Decke 23 ist eine Führungsöffnung 24 belassen. Diese mitteloder unmittelbar ausgeführte Führungsöffnung 24 nimmt als Herzstück der Dosiervorrichtung D einen Tauchschieber 25 auf. Dieser fungiert zufolge entsprechender Ausgestaltung als eine bewegte Dosierkammer 26 für die auszuhebende Teilmenge 10', wobei die Bewegung des Tauchschiebers 25 linear in der Längsmittelachse x-x des im Wesentlichen rotationssymmetrisch gestalteten Inhalators 1 erfolgt, überlagert von einer um diese Längsmittelachse x-x durchgeführten Rotationsbewegung. Der Tauchschieber 25 ist im Wesentlichen als Flachteil ausgeformt mit langgestreckt rechteckigem Querschnitt. Das Längen verhältnis von Schmalseite zu Breitseite beträgt in dem dargestellten Ausführungsbeispiel etwa 1:3.

An dem dem Mundstück 3 abgewandten Ende bildet der Tauchschieber 25 eine annähernd schraubendreherklingenartige Zuspitzung aus. Die zwei spiegelsymmetrischen Schrägflanken gehen hierbei von den jeweiligen Breitseiten des Tauchschiebers 25 aus. Das freie schrägflankenbesetzte Ende ist verstumpft.

Die Querschnittsausgestaltung des Tauchschiebers 25 und die Ausspitzung des freien Endbereichs haben aufgrund der Drehmitnahme des Tauchschiebers 25 im Zentrumsbereich auflockernde Wirkung in Bezug auf den See aus pulverförmiger Substanz 10.

Der Hubweg der linear sowie überlagert rotatorisch bewegten Dosierkammer 26 berücksichtigt in beiden Endstellungen des Tauchschiebers 25 ein Zuhalten des Querschnitts der Führungsöffnung 24 mit dosierkammerfüllender Rakel- bzw. Abstreichwirkung über die Länge der besagten Öffnung 24.

Das mundstückseitige Ende der Verschlusskappe 4 bildet eine bei Überlast ausklinkende Andockstelle 28 zwischen Tauchschieber 25 und Verschlusskappe 4. Das verschlusskappenseitige Rastmittel ist dabei ein ausfederfähiger Hakenkranz. Das korrespondierende Ende des Tauchschiebers 25 ist im Querschnitt rotationssymmetrisch ausgeformt, wobei weiter im Übergangsbereich vom Flachteilabschnitt zum zylinderförmigen Endabschnitt ein tellerförmiger Radialkragen 29 auswächst. Mit axialem Abstand zu diesem Radialkragen 29 formt der dem Flachteil abgewandte Endbereich des Tauchschiebers 25 einen Rastkopf 30 aus. Zwischen diesem und dem Radialkragen 29 ist eine wespentaillenartige Ringnut 31 gebildet. In diese greifen einwärts gerichtete Nasen 32 der federnden Zungen des Hakenkranzes ein. Der Rastkopf 30 ist in beiden Richtungen durch die Nasen 32 überwindbar.

Die Nasen 29 bzw. ihre Federzungen sind an einem in eine zentrale Mundstücköffnung 3' ragenden Röhrchen 33 realisiert, welches von der Deckeninnenseite der Verschlusskappe 4, an diesem wurzelnd ausgeht.

Das Mundstück 3 greift über eine Mantelwand 34 verankernd am Hals des Gehäuses 2 an. Diese Verankerung ist mit Bezug auf die Darstellungen unterhalb der Drehteil-Decke 23 in Form einer Raststelle 35 zwischen den beiden Teilen 2,3 ausgebildet. Es kann sich hierbei um eine irreversible Raststelle 35 handeln. Darüber hinaus ist die Decke 23 des Drehteiles 22 durch eine Ringschulter 36 der Mantelwand 34 abgestützt überfangen.

Die zentrale Öffnung 3' des Mundstückes 3 ist im Bereich eines im Wesentlichen in Überkopfstellung angeordneten, topfförmigen Dispergierteils 37 ausgebildet. Dies durch zentrale Durchsetzung des Dispergierteil-Bodens 38. Das in Richtung auf das Drehteil 22 sich hin öffnende Dispergierteil 37 weist eine Topfwandung 39 auf, mit einem Außendurchmesser, welcher dem Außendurchmesser der Topfwandung 40 des Drehteiles 22 entspricht. Das topfförmige Drehteil 22 und das topfförmige Dispergierteil 37 weisen mit ihren Öffnungen aufeinander zu, wobei das Dispergierteil 37 sich mit seiner freien Ringkante auf der zugeordneten Ringkante der Drehteil-Topfwandung 40 abstützt.

Beide Topfwandungen 39 und 40 sind radial nach innen beabstandet zur Innenwandung der Mantelwand 34. Entsprechend stellt sich jeweils um das Drehteil 22 und um das Dispergierteil 37 ein Ringraum 41 ein.

Der Innendurchmesser der Topfwandung 39 des Dispergierteiles 37 ist an den Außendurchmesser des tellerartigen Radialkragens 29 des Tauchschiebers 25 angepasst. Entsprechend erfährt letzterer in dem topfartigen Dispergierteil 37 eine Führung in Linearrichtung.

Jeweils zu ihren offenen Endbereichen hin erweitern sich die Topfräume sowohl des Drehteils 22 als auch des Dispergierteils 37 nach radial außen unter Materialreduzierung der jeweiligen Topfwandungen 39 und 40. Zufolge dieser Ausgestaltung stellt sich ein radial erweiterter Umströmungsbereich 42 ein.

Mit etwa der Materialstärke des Radialkragens 29 des Tauchschiebers 25 zum Topfboden 38 des Dispergierteils 37 beabstandet sind in der Topfwandung 39 Radialdurchlässe 43 vorgesehen, zur Verbindung des Topfinnenraumes mit dem umlaufenden Ringraum 41. Es können wie dargestellt zwei diametral gegenüberliegende Durchlassöffnungen 43 vorgesehen sein. Alternativ kann auch ein umlaufender, durch Stützstege unterbrochener Durchlassschlitz vorgesehen sein.

Der den Topfboden 38 des Dispergierteils 37 umfassende Austrags-Ringraum 44 ist zu dem in etwa in axialer Verlängerung sich erstreckenden Ringraum 41 durch einen an der Topfwandung 39 radial nach außen abragenden Dichtkragen 45 getrennt, welcher Dichtkragen 45 sich innenseitig der Mantelwandung 34 abstützt. Zufolge dieser Ausgestaltung ist eine definierte Umlenkung des Saugluftkanals 12 aus der zentralen axialen Ausrichtung nach radial außen in den im Wesentlichen axial ausgerichteten Auslass-Ringraum 44 erreicht.

Die axialen Längen von Drehteil 22 und Dispergierteil 37 im Bereich ihrer Topfwandungen 39 und 40 sind so gewählt, dass der pulverschöpfende Tauchhub des Tauchschiebers 25 aus einer Befüllungsebene in der Vorratskammer 11 bis in die Übergabestelle U oberhalb der Decke 23 gewährleistet ist.

Die definierte Entleerungsbereitschaftsstellung der Dosierkammer 26 ergibt sich durch Zugbegrenzungsanschlag des Tauchschiebers 25 im Bereich seines Radialkragens 29 gegen den Topfboden 38 des Dispergierteils 37.

Die Dosierkammer 26 ist als im Wesentlichen senkrecht zur Längsmittelachse x - x verlaufende Querbohrung realisiert.

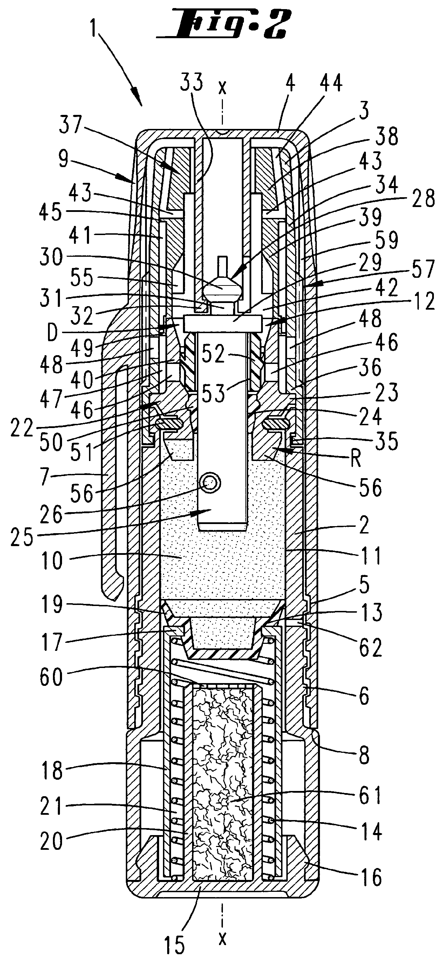

Diese Längsmittelachse x - x bildet zugleich die Rotationsachse. Mit Bezug zu dieser Rotationsachse ist die Dosierkammer 26 exzentrisch angeordnet, so weiter die Breitseiten des als Flachteil ausgeformten Tauchschiebers 25 durchsetzend. Wie insbesondere aus der Darstellung in Figur 2 zu erkennen, ist die Dosierkammer 26 mit Abstand zum freien Ende des Tauchschiebers 25 einer Seitenrandkante der Breitfläche zugeordnet angeordnet.

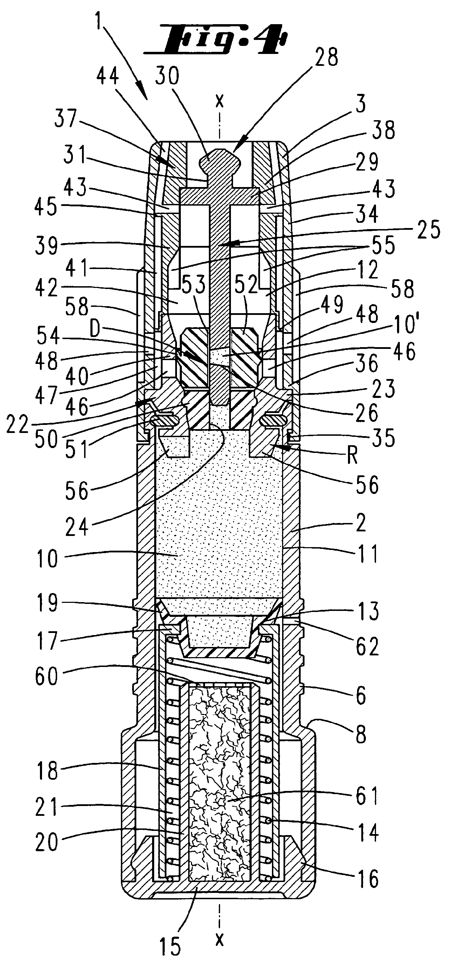

In der Entleerungsbereitschaftsstellung gemäß Figur 4 steht die Dosierkammer 26 im Wirkungsbereich des zentralen Saugluftstromes S. Der Dosierkammer 26 ist ein an den Saugluftkanal 12 anschließender Luftdurchlass 46 zugeordnet, welcher in der Topfwandung 40 des Drehteils 22 ausgebildet ist. Hierbei handelt es sich um radiale Bohrungen, die sich in Bodennähe des topfförmigen Drehteils 22 mit axialem Abstand oberhalb der Oberseite der Decke 23 erstrecken.

Beiden offenen Enden der Dosierkammer 26 ist ein solcher Luftdurchlass 46 mit radialem Abstand vorgelagert. In diesem Zusammenhang kann eine Vorkehrung darin bestehen, dass dem Ende größeren lichten Durchmessers der von einer konischen Querbohrung gebildeten Dosierkammer 26 ein Luftdurchlass 40 kleineren Durchmessers als dieser zugeordnet ist und dem Ende kleineren lichteren Durchmesser der Dosierkammer 26 ein Luftdurchlass 46 größeren Durchmessers als dieser. Hierdurch kann sich hinter dem Luftdurchlass 46 kleineren Durchmessers ein größerer Unterdruck mit vorrangiger Austragswirkung bezüglich der dargebotenen Teilmenge 10' ergeben. Gleichwohl findet der Austrag, das heißt die Entleerung der Dosierkammer 26 von beiden Enden her statt. In den Zeichnungen ist eine Lösung dargestellt, bei welcher die Luftdurchlässe 46 gleiche Durchmesser aufweisen.

Die am topfförmigen, den Tauchschieber 25 führenden Drehteil 22 ausgebildeten Luftdurchlässe 46 sind über einen rückwärtigen Einström-Ringraum 47 radial beabstandet noch mit Lufteinlässen 48 strömungsverbunden. Auch diese sind als Bohrungen ausgeführt und stellen den Anschluss an die Atmosphäre. Der Einström-Ringraum 47 ist zwischen der Außenseite der Topfwandung 40 des topfförmigen Drehteils 22 und der Innenseite der Mantelwand 34 des Mundstücks 3 ausgeformt, dies in axialer Verlängerung zum beschriebenen Ringraum 41. Ein abgestufter, nach radial außen ragender Dichtkragen 49 an dem der Topföffnung des Dispergierteils 37 zugewandten Ende dient der Trennung der Ringräume zueinander sowie zur radialen Ausrichtung des Dispergierteils 37 unter Abstützung an der Innenseite der Mantelwand 34. Die Dichtkragen 49 und 45 des Dispergierteils 37 verhindern einen Strömungskurzschluss zwischen den Lufteinlässen 48 und dem Auslass-Ringraum 44 im Bereich des Mundstückes 3.

Die Luftdurchlässe 46 sind axial versetzt zu den Lufteinlässen 48 angeordnet, wobei letztere dem Mundstück 3 näher liegen. Die beschriebene räumliche Abstandslage führt zu einer zunächst gegenläufigen Einströmung eingesaugter Luft mit Anschluss an den Haupt-Saugluftstrom S.

Die Führungsöffnung 24 für den Tauchschieber 25 ist abstreifend ausgebildet, zufolge dessen auch kein an dem Tauchschiebermantel etwa anhaftendes Pulvergut dosisverfälschend mitgeschleppt wird. Die Führungsöffnung 24 ist nicht unmittelbar vom Drehteil 22 gebildet, sondern durch eine diese Durchtrittsstelle auskleidende Dichtbuchse 50. Letztere besteht aus gummielastischem Material und ist in die Decke 23 über Rastmittel eingeklipst gehalten.

Zwischen dem Drehteil 22 und dem die Vorratskammer 11 bildenden Gehäuse 2 befindet sich gleichfalls ein Dichtelement. Erreicht ist dies durch einen zwischen die Innenwand der Vorratskammer 11 und dem Drehteil 22 eingesetzten Dichtring 51 aus gummielastischem Material. Letzterer ist unter Vorspannung in Ringnuten beider Teile 2, 22 passend eingesetzt. Beide den Dichtring 51 aufnehmende umlaufende Ringnuten weisen eine halbrunde Querschnittsgestaltung auf. Die korrespondierenden Bereiche des Dichtringes 51 sind entsprechend geformt.

Die Dichtbuchse 50 ist drehfest mit dem Drehteil 22 verbunden. Die Führungsöffnung 24 ist angepasst an die Querschnittsausgestaltung des Tauchschiebers 25 gleichfalls langgestreckt rechteckig ausgebildet, zufolge dieses Formschlusses auch der Tauchschieber 25 mit dem Drehteil 22 drehfest verbunden ist.

Mit dem Tauchschieber 25 wirkt ein relativ zu diesem außerhalb der Vorratskammer 11 beweglicher Verschlusskolben 52 zusammen. Dieser kann aus einem gummielastischen Material bestehen und ist zentral durchsetzt vom Flachteilabschnitt des Tauchschiebers 25, wozu der Verschlusskolben 52 eine angepasste, im Grundriss langgestreckt rechteckförmige Lageröffnung 53 besitzt. Diese Lageröffnung 53 ist mit Bezug auf die Querschnittsabmessung des Flachteilabschnittes des Tauchschiebers 25 leicht vergrößert, zufolge dessen eine reibungsarme Verlagerung des Verschlussstopfens 52 auf dem Tauchschieber 25 erreicht ist.

Der Verschlusskolben 52 ist mit einer radial außen umlaufenden Dichtlippe 54 versehen, welche in einer Übergabebereitschaftsstellung gemäß der Darstellung in Figur 4 mit der Innenwand der Drehteil-Topfwandung 40 zusammenwirkt, dies mit Bezug zu der Vorratskammer 11 oberhalb der Luftdurchlässe 46.

In dieser Übergabe-Bereitschaftsstellung liegt der Verschlussstopfen 52 sperrend im Saugluftkanal 12 unter beidseitiger Abdichtung des mit der Teilmenge 10' der Substanz 10 versehenen Dosierkammer 26. Ein Ablegen des Inhalators 1 nach Aktivierung desselben, das heißt nach Verlagerung des Tauchschiebers 25 mit der befüllten Dosierkammer 26 in die Übergabe-Bereitschaftsstellung hat zufolge der Anordnung des Verschlusskolbens 52 nicht den Verlust der abgeteilten Dosiermenge aus der Dosierkammer 26 zur Folge. Der Verschlussstopfen 52 ist selbsthemmend ausgeformt.

Die Verlagerung des Verschlusskolbens 52 ist nur willensbetont möglich, dies in einfachster Weise durch Aktivierung des Saugluftstromes S, das heißt durch üblichen Inhalationssog. In Abhängigkeit von dem hierbei entstehenden Saugunterdruck wird der Verschlusskolben 52 entlang des Flachteilabschnitts des Tauchschiebers 25 nach axial oben verlagert, zur beidseitigen Freigabe der Dosierkammer 26. Der Verschlusskolben 52 wird hierbei in den radial erweiterten Umströmungsbereich 42 bewegt, wobei diese axiale Verlagerung anschlagbegrenzt ist. Hierzu dienen axial ausgerichtete, von der Innenwandung der Dispergierteil-Topfwandung 39 radial nach innen sich erstreckende Anschlagstege 55, gegen welche die ringförmige Dichtlippe 54 des Verschlusskolbens 52 sperrend tritt. Der radiale Abstand der Anschlagstege 55 zueinander entspricht dem Führungsquerschnitt des Dispergierteiles 37 und somit dem Außendurchmesser des tauchschieberseitigen Radialkragens 29.

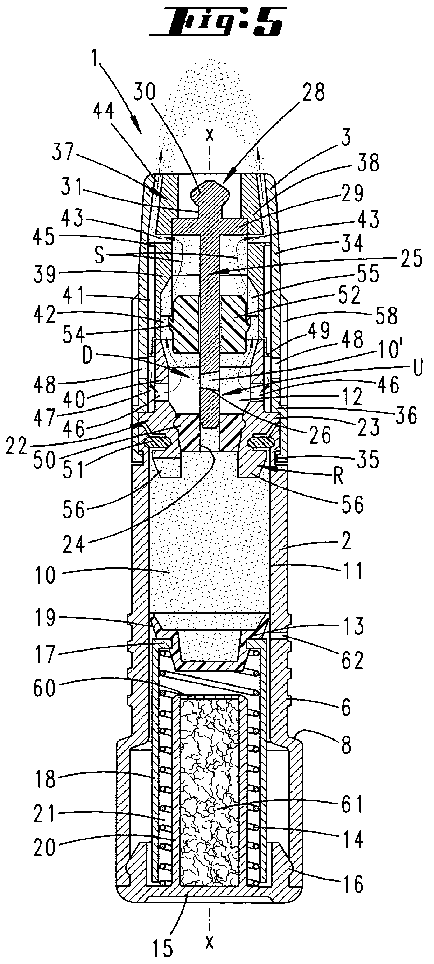

Im Zuge der - wie in Figur 5 schematisch dargestellten - Inhalation wird der Verschlusskolben 52 im Bereich 42 des Mundstückes 3 liegend umströmt.

Förderlich für das Entleeren der Vorratskammer 11 ist die Bereithaltung der pulverförmigen Substanz 10 im Schöpfbereich. Es sind Bedingungen geschaffen, die eine strukturgleiche bzw. homogene Befüllung der Dosierkammer 26 sicherstellen, gespeist aus einem durchgelockerten Umfeld. Hierzu ist vor allem das Drehteil 22 herangezogen. Dieses weist einen im oberen Bereich der Vorratskammer 11 agierenden Rotor R auf. Unter Nutzung der Rotation des Drehteils 22 ergibt sich ein Auflockern der bevorrateten Substanz. Schaufelbildend sind Rotorblätter 56. Diesbezüglich können zwei Rotorblätter 56 vorgesehen sein, welche mit Bezug auf die Längsmittelachse x - x des Inhalators 1 in diametraler Gegenüberlage angeordnet sind. Die vom Boden bzw. der Decke 23 des Drehteils 22 vorratskammerseitig abragenden, freistehenden Rotorblätter 56 sind in einer solchen diametralen Gegenüberlage positioniert, dass sie in Umlaufrichtung genügend beabstandet sind. Geometrisch können diese im Wesentlichen einen Viertelsektor des kreisrunden Querschnitts der Vorratskammer 11 einnehmen.

In Zusammenwirkung mit dem als Flachteil ausgeformten Tauchschieber 25 ist durch die Anordnung der Rotorblätter 56 stets ein gelockertes Umfeld erreicht. Weiter ist durch die zur Rotationsachse des Tauchschiebers 25 exzentrische Anordnung der Dosierkammer 26 eine optimale Befüllung derselben mittels schraubengangförmigen Durchtauchens, durch den Substanzsee erreicht.

Die Drehmitnahme zwischen Mundstück 3 und der sich schraubabhebenden Verschlusskappe 4 erfolgt durch eine Klauenkupplung 57 zwischen beiden. Die besteht aus einer Längszahnung 58 an der Mantelwand 34 des Mundstücks 3, welche Längszahnung 58 in korrespondierende Zahnlücken 59 an der Innenseite der Verschlusskappe 4 eingreifen.

Im Zuge des Schraubabhebens der Verschlusskappe 4 folgt über die Klauenkupplung 57 eine Drehmitnahme des Drehteils 22 und der hierüber mitgeschleppten Teile wie Dichtbuchse 50, Verschlussstopfen 52 und Tauchschieber 25, wobei weiter durch die schraubabhebende Verlagerung der Verschlusskappe 4 zugleich eine axiale Verlagerung des Tauchschiebers 25 über die Andockstelle 28 erfolgt, was eine schraubengangartige Verlagerung der Dosierkammer 26 in die Übergangsstelle U, das heißt in die Übergabe-Bereitschaftsstellung gemäß der Darstellung in Figur 4 bewirkt.

Der Verschlussstopfen 52 verbleibt im Zuge der Linearverlagerung des Tauchschiebers 25 in seiner sich auf der Decke 23 des Drehteils 22 abstützenden, dichtenden Stellung.

Der an der Bodenkappe 15 des Gehäuses 2 wurzelnde Stehzapfen 20 ist endseitig durch einen siebartigen Deckel 60 verschlossen. In dem hierdurch geschaffenen abgegrenzten Raum ist ein Feuchtigkeit absorbierendes Material 61 gefasst.

Der Tauchschieber 25 kann bezüglich des Volumens seiner Dosierkammer 26 variiert werden. Hierzu braucht das Herzstück der Dosiervorrichtung D nämlich der Tauchschieber 25 lediglich ausgetauscht zu werden, um eine andere, genau reproduzierbare Dosierung von Teilmengen 10' zu erzielen.

Der kolbenartig wirkende Druckboden 13 wird in seiner Beweglichkeit gegenüber dem Zylinderraum, welcher vom mittleren Abschnitt des Gehäuses 2 gestellt wird, nicht beeinträchtigt, da das Gehäuse dort eine im Rücken der Ringlippe 19 liegende Luftausgleichsöffnung 62 besitzt.

Der topfförmige Druckboden 13 weist eine zentrale, der Vorratskammer 11 abgewandte Einziehung auf. Diese ist innen von solcher Tiefe, dass der in Grundstellung die Rotorblätter 56 axial nach unten überragende Endabschnitt des Tauchschiebers 25 darin unterkommt.

## Patentansprüche

1. Inhalator (1) für pulverförmige, insbesondere medizinische Substanzen, mit einem zu einem Mundstück (3) führenden Saugluftkanal (12), ferner einer Vorratskammer (11) für die Substanz (10) und einer linear sowie überlagert rotatorisch bewegten Dosierkammer (26) zum Abteilen einer bestimmten Substanzmenge (10') aus der Vorratskammer (11) und Verbringen der Substanzmenge (10') in eine Übergabeposition, zur Übergabe an den Saugluftstrom (S), **dadurch gekennzeichnet, dass** die Dosierkammer (26) in einem als Flachteil ausgeformten Tauchschieber (25) ausgebildet ist und exzentrisch zu der Rotationsachse (x) ihrer überlagerten rotatorischen Bewegung angeordnet ist.

2. Inhalator nach Anspruch 1, **dadurch gekennzeichnet, dass** der Tauchschieber (25) außerhalb der Vorratskammer (11) mit einem relativ zu dem Tauchschieber beweglichen Verschlusskolben (52) zusammenwirkt.

3. Inhalator nach Anspruch 2, **dadurch gekennzeichnet, dass** der Verschlusskolben (52) abhängig von einem Saugunterdruck in eine Freigabestellung bezüglich der Dosierkammer (26) zu bewegen ist.

4. Inhalator nach einem der vorangehenden Ansprüche, **dadurch gekennzeichnet, dass** der Saugluftkanal (12) oberhalb der Dosierkammer (26) eine Umlenkung nach radial außen aufweist

5. Inhalator nach Anspruch 4, **dadurch gekennzeichnet, dass** der Tauchschieber (25) selbst einen Teil der Strömungsumlenkung ausbildet.

## Claims

1. Inhaler (1) for powdered, particularly medical substances, with a suction air channel (12) leading to a mouthpiece (3), also a storage chamber (11) for the substance (10) and a linearly moved dosing chamber (26), which is also rotationally moved in a superposed manner, for apportioning a specific amount (10') of substance from the storage chamber (11) and bringing the amount (10') of substance into a transfer position, for transfer to the suction air stream (S), **characterized in that** the dosing chamber (26) is formed in a plunger slide (25) fashioned as a flat part and is disposed eccentrically in relation to the axis of rotation (x) of its rotational movement in a superposed manner.

2. Inhaler according to Claim 1, **characterized in that** the plunger slide (25) interacts outside the storage chamber (11) with a closure plunger (52) that is movable in relation to the plunger slide.

3. Inhaler according to Claim 2, **characterized in that** the closure plunger (52) can be moved into a release position with respect to the dosing chamber (26) in dependence on a negative suction pressure.

4. Inhaler according to one of the preceding claims, **characterized in that** the suction air channel (12) has a radially outward deflection above the dosing chamber (26).

5. Inhaler according to Claim 4, **characterized in that** the plunger slide (25) itself forms part of the flow deflection.

## Revendications

1. Inhalateur (1) pour substances pulvérulentes, en particulier pour substances médicales, avec un canal d'air d'aspiration (12) conduisant à un embout buccal (3), avec en outre une chambre de stockage (11) pour la substance (10) et avec une chambre de dosage (26) déplacée linéairement ainsi que de façon superposée en rotation pour diviser une quantité déterminée de substance (10') hors de la chambre de stockage (11) et amener la quantité de substance (10') dans une position de transfert, pour le transfert au flux d'air d'aspiration (S), **caractérisé en ce que** la chambre de dosage (26) est formée dans un plongeur coulissant (25) réalisé en forme de partie plate et est disposée de façon excentrique par rapport à l'axe de rotation (x) de son mouvement de rotation superposé.

2. Inhalateur selon la revendication 1, **caractérisé en ce que** le plongeur coulissant (25) coopère à l'extérieur de la chambre de stockage (11) avec un piston de fermeture (52) mobile par rapport au plongeur coulissant.

3. Inhalateur selon la revendication 2, **caractérisé en ce que** le piston de fermeture (52) doit être déplacé en fonction d'une dépression d'aspiration dans une position de libération par rapport à la chambre de dosage (26).

4. Inhalateur selon l'une quelconque des revendications précédentes, **caractérisé en ce que** le canal d'air d'aspiration (12) présente au-dessus de la chambre de dosage (26) une déviation vers l'espace radialement extérieur.

5. Inhalateur selon la revendication 4, **caractérisé en ce que** le plongeur coulissant (25) forme lui-même une partie de la déviation d'écoulement.
